# EUROPEAN PATENT APPLICATION

(11) **EP 2 436 385 A1**
(43) Date of publication of application: **04.04.2012**
(21) Application number: 09845227.9
(22) Date of filing: 29.05.2009
(51) Int. Cl.: A61K 31/505, A61P 11/00, C07D 239/34

(54) **PROPHYLACTIC OR THERAPEUTIC AGENT FOR PULMONARY FIBROSIS**

(71) Applicant: Shizuoka Coffein Co., Ltd., Shizuoka-shi, Shizuoka 420-0008 (JP)
(72) Inventor: HATTORI Noboru, Hiroshima-shi Hiroshima 734-8551 (JP); UEDA Kentaro, Shizuoka-shi Shizuoka 420-0007 (JP); AKASHIMA Makoto, Shizuoka-shi Shizuoka 422-8007 (JP); TAKAGI Masamichi, Shizuoka-shi Shizuoka 421-0101 (JP)
(74) Representative: Duckworth, Timothy John
(86) International application number: PCT/JP2009/059840
(87) International publication number: WO 2010/137160

(57) **Abstract**

Disclosed is a compound which has an inhibitory activity on plasminogen activator inhibitor-1 (PAI-1) and has a propanedioic acid structure represented by formula (1) [wherein R₁ and R₂ independently represent a hydrogen atom or a linear or branched alkyl group having 1 to 4 carbon atoms; R₃ represents a cyclohexyl group or a phenyl group (provide that the phenyl group may be substituted by a substituent selected from a linear or branched alkyl group having 1 to 4 carbon atoms, a linear or branched alkoxy group having 1 to 4 carbon atoms, and a halogen atom); R₄ represents a linear or branched alkyl group having 1 to 6 carbon atoms or a phenyl group (provide that the phenyl group may be substituted by a substituent selected from a linear or branched alkyl group having 1 to 4 carbon atoms, a linear or branched alkoxy group having 1 to 4 carbon atoms, and a halogen atom); and n represents an integer of 1 to 8.. The compound is useful as a prophylactic or therapeutic agent for pulmonary fibrosis.

## Description

### Technical Field

The present invention relates to a prophylactic or therapeutic agent for pulmonary fibrosis comprising a propanedioic acid derivative having plasminogen activator inhibitor-1 (PAI-1) inhibitory activity as an active ingredient.

### Background Art

"Pulmonary fibrosis" generally refers to a disease group in which alveolar tissue is destroyed by inflammatory reaction and as a result, the lung is hardened due to a remodeled lesion in the alveolar region caused by the growth of fibroblasts and the excessive hyperplasia of extracellular matrix composed mainly of collagen, and thereby loses its functions. It is also known that the advanced stage of pulmonary fibrosis causes cyanosis attributed to reduced oxygen concentrations in blood, dyspnea or respiratory failure, or cardiac failure and is further complicated with lung cancer or the like with high frequency. Examples of causes of the onset of pulmonary fibrosis include inhalation of inorganic or organic particles of dust, adverse drug reaction, radiotherapy, infection, and collagen diseases such as rheumatoid arthritis and connective tissue disease. Moreover, pulmonary fibrosis whose cause of the onset is unidentified is called "idiopathic pulmonary fibrosis", which is a disease resulting in exceedingly poor prognosis such that the average survival period after its onset is 4 to 5 years. This disease is designated as an intractable disease (specified disease) in Japan.

Currently, a method involving the administration of an anti-inflammatory agent (e.g.; steroid) or an immunosuppressive agent is adopted as a method for treating pulmonary fibrosis and is however ineffective in many times. Moreover, "Pirespa 200 mg Tablet (generic name: pirfenidone)" has been approved as a therapeutic agent for idiopathic pulmonary fibrosis in Japan and is however known to cause photosensitivity as adverse reaction with high frequency. Thus, there is a demand for the development of a safer and more highly effective drug.

In recent years, a method using a plasminogen activator inhibitor-1 (PAI-1) inhibitor has been studied as one of methods for treating pulmonary fibrosis. PAI-1 inhibits tissue plasminogen activator (t-PA) and urokinase plasminogen activator (u-PA) and suppresses plasmin formation, thereby inhibiting fibrin degradation. Furthermore, PAI-1 promotes the deposition and accumulation of extracellular matrix and is deeply involved in the progression of tissue lesions including fibrosis or hardened lesions in blood vessel walls. Therefore, the PAI-1 inhibitor is expected as a drug effective for the treatment of blood vessel disease such as thrombosis or fibrotic disease such as pulmonary fibrosis.

For example, Non Patent Literatures 1 and 2 have reported as to the relation of pulmonary fibrosis to PAI-1 that in a test using mouse models with bleomycin-induced pulmonary fibrosis, the progression of bleomycin-induced pulmonary fibrosis is prevented in PAI-1 gene-deficient mice compared with wild-type mice.

Moreover, in Non Patent Literature 2, polyvinyl alcohol sponge was subcutaneously transplanted to PAI-1 gene-deficient mice and wild-type mice, and the accumulation of fibrous tissue was compared therebetween. As a result, it has been reported that the invasion of fibrous tissue is more delayed in the PAI-1 gene-deficient mice.

On the other hand, Non Patent Literatures 1 and 3 have shown that in fibrinogen-knockout mice after bleomycin treatment, pulmonary fibrosis progresses to the same degree as in control mice, and have reported that fibrin is not essential for the progression of pulmonary fibrosis.

Patent Literatures 1 and 2 disclose that PAI-1 inhibitors represented by following formulas (3) and (4), respectively, are useful for the prevention or treatment of pulmonary fibrosis: 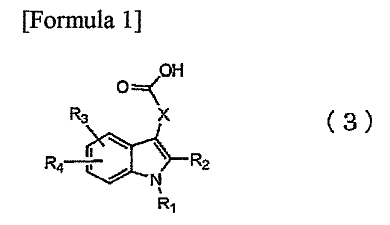 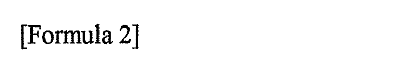 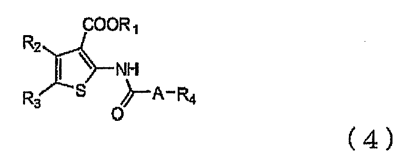 Particularly, Patent Literature 2 discloses that when the forced oral administration (200 mg/kg) of the PAI-1 inhibitor represented by the formula (4) was performed twice a day over the whole period from the day of bleomycin administration using mouse models with bleomycin-induced pulmonary fibrosis, this PAI-1 inhibitor was useful for the prevention or treatment of pulmonary fibrosis. However, there is a demand for a more highly effective PAI-1 inhibitor that is useful for the prevention or treatment of pulmonary fibrosis.

Patent Literatures 3 and 4 and Non Patent Literature 4 disclose that a propanedioic acid derivative has plasminogen activator (PA) activity promoting effect and PAI-1 inhibitory activity and is useful as a thrombolytic agent or an antithrombotic agent. Moreover, Patent Literature 4 and Non Patent Literature 4 disclose that a propanedioic acid derivative is also useful as an antineoplastic agent. However, the effectiveness of these propanedioic acid derivatives for pulmonary fibrosis has not been studied by any means.

### Citation List

### Patent Literature

| | |
|---|---|
| Patent Literature 1: | JP 2004-534817 A |
| Patent Literature 2: | International Publication No. WO 2007/083689 |
| Patent Literature 3: | International Publication No. WO 2004/011442 |
| Patent Literature 4: | International Publication No. WO 2007/138705 |

### Non Patent Literature

| | |
|---|---|
| Non Patent Literature 1: | The Journal of Clinical Investigation, Vol. 106, No. 11, Page. 1341-1350 (2000) |
| Non Patent Literature 2: | American Journal of Pathology, Vol. 163, No. 2, Page. 445-452 (2003) |
| Non Patent Literature 3: | American Journal of Pathology, Vol. 157, No. 3, Page. 703-708 (2000) |
| Non Patent Literature 4: | Carcinogenesis, Vol. 29, No. 4, Page. 824-829 (2008) |

### Summary of the Invention

### Problem to be solved by the Invention

An object of the present invention is to provide a prophylactic or therapeutic agent for pulmonary fibrosis comprising a propanedioic acid derivative having PAI-1 inhibitory activity.

### Means for Solving the Problem

Patent Literatures 3 and 4 and Non Patent Literature 4 disclose that a propanedioic acid derivative has PA activity promoting effect and PAI-1 inhibitory activity. Moreover, Patent Literature 4 and Non Patent Literature 4 disclose that a propanedioic acid derivative is also useful as an antineoplastic agent. Nevertheless, the effectiveness of these propanedioic acid derivatives for pulmonary fibrosis has not been studied by any means.
Thus, focusing on the relation of PAI-1 to pulmonary fibrosis, the present inventors have conducted diligent studies on the development of a novel prophylactic or therapeutic agent for pulmonary fibrosis comprising a propanedioic acid derivative having PAI-1 inhibitory activity and completed the present invention.
Specifically, the present invention relates to a pharmaceutical composition for preventing or treating pulmonary fibrosis, comprising a propanedioic acid derivative represented by a following formula (1) or a pharmaceutically acceptable salt thereof as an active ingredient: 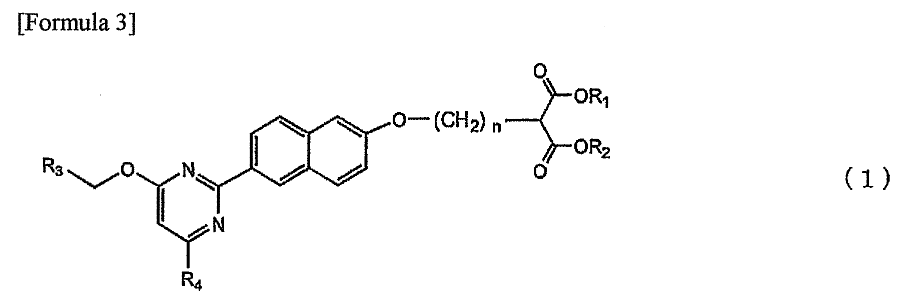 wherein
R₁ and R₂ each independently represent a hydrogen atom or a linear or branched alkyl group having 1 to 4 carbon atoms;
R₃ represents a cyclohexyl group or a phenyl group (wherein the phenyl group may be substituted by a substituent selected from a linear or branched alkyl group having 1 to 4 carbon atoms, a linear or branched alkoxy group having 1 to 4 carbon atoms, and a halogen atom);
R₄ represents a linear or branched alkyl group having 1 to 6 carbon atoms or a phenyl group (wherein the phenyl group may be substituted by a substituent selected from a linear or branched alkyl group having 1 to 4 carbon atoms, a linear or branched alkoxy group having 1 to 4 carbon atoms, and a halogen atom); and
n represents an integer of 1 to 8.
The present invention further relates to use of the propanedioic acid derivative represented by the formula (1) or the pharmaceutically acceptable salt thereof for producing a prophylactic or therapeutic agent for pulmonary fibrosis.
The present invention further relates to a method for preventing or treating pulmonary fibrosis, comprising administering the propanedioic acid derivative represented by the formula (1) or the pharmaceutically acceptable salt thereof to a human or a nonhuman mammal.

### Advantages of the Invention

In a test using mouse models with bleomycin-induced pulmonary fibrosis, a propanedioic acid derivative of the present invention significantly suppressed particularly the fibrotic process in the lung during the course of an inflammatory stage for several days after bleomycin administration and a subsequent pulmonary fibrotic stage. Thus, the compound of the present invention exerts particularly excellent effect as a prophylactic or therapeutic agent for pulmonary fibrosis. Furthermore, when an aqueous solution containing approximately 1000 ppm propanedioic acid derivative of the present invention dissolved therein was administered as drinking water to mouse models with bleomycin-induced pulmonary fibrosis, it significantly suppressed particularly the fibrotic process in the lung. Thus, the propanedioic acid derivative of the present invention can exhibit effect even at a low dose and exerts particularly excellent effect as a prophylactic or therapeutic agent for pulmonary fibrosis. Moreover, the propanedioic acid derivative of the present invention has high solubility in water and also high light resistance, is easily formulated, and can form a stable preparation. Thus, the propanedioic acid derivative of the present invention is excellent in various points.

### Brief Description of Drawings

[Fig. 1] Fig. 1 shows an administration schedule in Example 4. A period for 7 days after bleomycin administration is defined as an early stage (period in which inflammation is induced: inflammatory stage), and a period from 8 days to 20 days after bleomycin administration is defined as a late stage (period in which fibrosis is induced: fibrotic stage). An aqueous solution containing 1000 ppm 2Na salt of a compound (1) dissolved therein was administered as drinking water.
[Fig. 2] Fig. 2 is a graph showing a PAI-1 concentration (ng/mL) in a bronchoalveolar lavage fluid (BALF) in Example 4.
[Fig. 3] Fig. 3 is a graph showing the amount of hydroxyproline in Example 4. In group 2 (late stage), the amount of hydroxyproline was significantly decreased compared with a control group. (*: p < 0.05)

### Mode for Carrying Out the Invention

Hereinafter, the embodiments of the present invention will be described in detail.
In the present invention, specific examples of a "linear or branched alkyl group having 1 to 4 carbon atoms" include methyl, ethyl, propyl, isopropyl, butyl, isobutyl, sec-butyl, and tert-butyl groups.
Specific examples of a "linear or branched alkyl group having 1 to 6 carbon atoms" include methyl, ethyl, propyl, isopropyl, butyl, isobutyl, sec-butyl, tert-butyl, pentyl, isopentyl, neopentyl, tert-pentyl, hexyl, isohexyl, and neohexyl groups.
Specific examples of a "linear or branched alkoxy group having 1 to 4 carbon atoms" include methoxy, ethoxy, propoxy, isopropoxy, butoxy, isobutoxy, sec-butoxy, and tert-butoxy groups.
Specific examples of a "halogen atom" include chlorine, bromine, and fluorine atoms.

A propanedioic acid derivative represented by the formula (1) of the present invention wherein R₃ represents a phenyl group and R₄ represents a phenyl group is preferably a propanedioic acid derivative represented by a following formula (2): 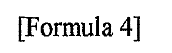 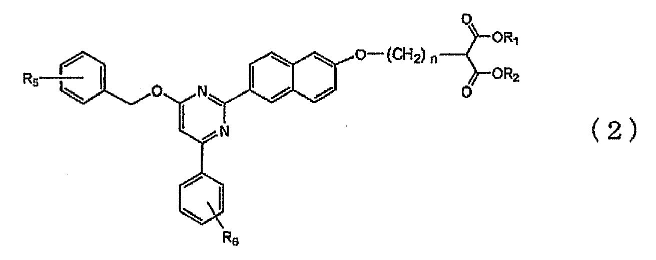 wherein R₁, R₂, and n are as defined above; R₅ represents a hydrogen atom or a halogen atom; and R₆ represents a hydrogen atom or a linear or branched alkoxy group having 1 to 4 carbon atoms.

Preferable specific examples of the propanedioic acid derivative represented by the formula (1) of the present invention include following compounds:
(1) [5-[[6-[4-phenyl-6-(phenylmethoxy)-2-pyrimidinyl]-2-naphthalenyl]oxy]pentyl]propanedioic acid,
(2) [3-[[6-[4-[(2-fluorophenyl)methoxy]-6-phenyl-2-pyrimidinyl]-2-naphthalenyl]oxy]propyl]propanedioic acid,
(3) [3-[[6-[4-[(2-fluorophenyl)methoxy]-6-(4-methoxyphenyl)-2-pyrimidinyl]-2-naphthalenyl]oxy]propyl]propanedioic acid,
(4) [5-[[6-[4-(cyclohexylmethoxy)-6-(1,1-dimethylethyl)-2-pyrimidinyl]-2-naphthalenyl]oxy]pentyl]propanedioic acid,
(5) [5-[[6-[4-(1,1-dimethylethyl)-6-[(4-fluorophenyl)methoxy]-2-pyrimidinyl]-2-naphthalenyl]oxy]pentyl]propanedioic acid, and
(6) [5-[[6-[4-[(2-chlorophenyl)methoxy]-6-(1,1-dimethylethyl)-2-pyrimidinyl]-2-naphthalenyl]oxy]pentyl]propanedioic acid.

The structures of these compounds are shown in Table 1.

### [Table 1]

**Table 1 Structural formulas of propanedioic acid derivatives**

| Compound No. | Structural formula |
|---|---|
| (1) | 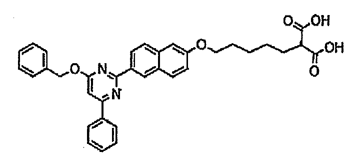 |
| (2) | 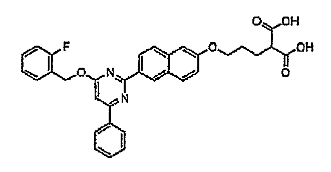 |
| (3) | 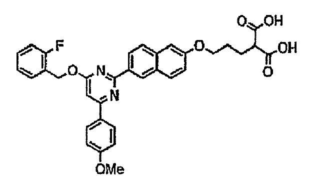 |
| (4) | 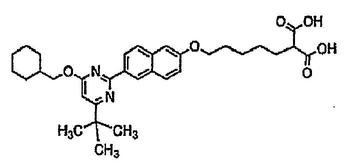 |
| (5) | 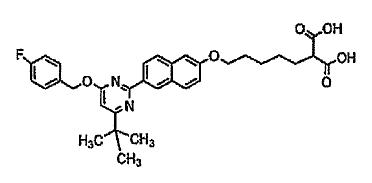 |
| (6) | 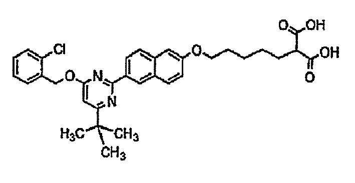 |

Any of the propanedioic acid derivatives of the present invention are compounds known in the art. These can be produced according to a method described in Patent Literature 3. However, the production method is not limited to this, and they can also be produced by a production method well known by those skilled in the art.

The propanedioic acid derivative of the present invention may be a pharmaceutically acceptable salt. In this context, the "pharmaceutically acceptable salt" refers to a salt that is prepared from a pharmaceutically acceptable nontoxic base or acid. The salt of the propanedioic acid derivative represented by the formula (1), which is preferably used in the present invention, can be prepared from a pharmaceutically acceptable nontoxic base including inorganic and organic bases. The salts derived from the inorganic bases encompass potassium salt, sodium salt, calcium salt, magnesium salt, aluminum salt, ammonium salt, and the like. Potassium salt and sodium salt are particularly preferable.

The salts derived from the pharmaceutically acceptable organic nontoxic bases encompass primary amine salt, secondary amine salt, tertiary amine salt, and cyclic amine salt, and the like.

Moreover, the salt of the propanedioic acid derivative represented by the formula (1) can be prepared from a pharmaceutically acceptable nontoxic acid including inorganic and organic acids. Examples of the nontoxic acids include acetic acid, citric acid, succinic acid, tartaric acid, maleic acid, methanesulfonic acid, benzenesulfonic acid, toluenesulfonic acid, hydrochloric acid, sulfuric acid, and phosphoric acid.

When the propanedioic acid derivative of the present invention is administered, it may be administered alone and however, is generally administered in the form of a preparation usually used, together with a pharmaceutically acceptable carrier and/or excipient.

The "pharmaceutically acceptable carrier" may be in a solid, liquid, or gas state. Examples of the solid carrier include acacia, agar, gelatin, talc, and lactose. Examples of the liquid carrier include water, olive oil, and sugar syrup. Examples of the gas carrier include nitrogen.

The propanedioic acid derivative of the present invention can usually be administered to a mammal (including humans) orally, parenterally, percutaneously, by injection, by inhalation or using a spray, sublingually, transrectally, or transvaginally.

The preparation for oral administration can be produced as tablets or capsules according to a routine method by adding, if necessary, a pharmacologically or pharmaceutically acceptable excipient or the like together with the pharmaceutically acceptable carrier described above.

For example, a diluent (e.g., magnesium aluminometasilicate, magnesium silicate, magnesium carbonate, calcium hydrogen phosphate, various starches, and dextrin), a binder (e.g., ethylcellulose (EC), hydroxypropylmethylcellulose (HPMC), hydroxypropylcellulose (HPC), sodium alginate, and gelatin), a corrigent (e.g., mannitol, citric acid, sodium citrate, and sugar), and other excipients such as a disintegrant, a wetting agent, and a coating agent can be used in the oral preparation.

The oral liquid preparation may be in the form of an aqueous or oil suspension, a solution, an emulsion, a syrup, or the like and can contain, for example, a suspending agent such as methylcellulose, carboxymethylcellulose, sodium alginate, gelatin, or propylene glycol, a sweetener such as sucrose or saccharine, or an emulsifying agent such as gelatin, macrogol (PEG), or propylene glycol fatty acid ester.

The administration by injection means intravenous, intraarterial, intramuscular, and subcutaneous injections, and the like. An injection containing the propanedioic acid derivative of the present invention can be prepared as a sterile aqueous solution. Furthermore, it can be prepared in the form of sterile powder to be prepared into an injectable solution.

For example, water as a solubilizer or ethanol, glycerol, propylene glycol, or the like as a solubilization aid can be used as a carrier for injections. In addition, pharmaceutical ingredients such as a pH adjuster, a tonicity agent, and a stabilizer can also be used.

Moreover, the propanedioic acid derivative of the present invention can be prepared as a suppository for rectal administration. For the suppository, an appropriate substance such as cacao butter, laurin butter, macrogol, glycerogelatin, sodium stearate, or a mixture thereof is used as a base, to which an emulsifying agent, a suspending agent, a preservative, and the like can be added, if necessary.

Examples of inhalants include aerosols, powders for inhalation, liquid preparations for inhalation (e.g., solutions for inhalation and suspensions for inhalation), and inhalation capsules. The liquid preparations for inhalation may be dissolved or suspended in water or other appropriate media in use. These inhalants can be applied using an appropriate inhaler. For example, an atomizer or the like can be used for administering the liquid preparations for inhalation, and inhalers or the like for powdery drugs can be used for administering the powders for inhalation. Moreover, an appropriate excipient may be blended, if necessary, in these inhalants. A diluent, a binder, a lubricant, a preservative, a stabilizer, and the like can be used as excipients.

The propanedioic acid derivative of the present invention can be administered through any of the administration routes described above and can be administered at a dose ranging from 0.1 to 300 mg/kg to a patient. Furthermore, the propanedioic acid derivative of the present invention can be administered once or more a day. These numeric values are provided strictly for the illustrative purpose, and the dose is appropriately increased or decreased depending on various conditions such as the symptom of a patient.

The propanedioic acid derivative of the present invention was excellent in solubility in water in a solubility test. Furthermore, its aqueous solution was stable in a stability test. Thus, the propanedioic acid derivative of the present invention can be used easily as a raw material for the preparations described above and is very advantageous for the formulation of, for example, oral preparations, injections, and inhalants.

Hereinafter, the present invention will be described specifically with reference to Examples. However, the present invention is not intended to be limited to these Examples, etc., by any means.

### Example 1

### Solubility of propanedioic acid derivative of the present invention

Solubility test on a 2Na salt of a typical compound (1) (disodium salt of [5-[[6-[4-phenyl-6-(phenylmethoxy)-2-pyrimidinyl]-2-naphthalenyl]oxy]pentyl]propanedioic acid) used in the present invention in water is shown below.

### 1. Test method

Water of 20 ± 2°C was added to the 2Na salt of the compound (1), and the mixture was vigorously shaken for 30 seconds and left standing for 5 minutes. This operation was repeated until the 2Na salt of the compound (1) was completely dissolved. The final amount of water required for the dissolution was determined. The solubility of the 2Na salt of the compound (1) in water of 45 ± 5°C was determined by similar operation.

### 2. Results

The solubility of the 2Na salt of the compound (1) in water is shown in Table 2. The 2Na salt of the compound (1) was excellent in solubility in water.

**[Table 2]**

| Table 2 Solubility of 2Na salt of compound (1) in water | |
|---|---|
| Solvent | Amount of solvent required for dissolving 1 g (mL) |
| Water | 33 |
| (20±2°C) | |
| Water | 1.2 |
| (45±5°C) | |

### Example 2

### Stability of propanedioic acid derivative of the present invention in aqueous solution

Stability test on the 2Na salt of the compound (1) used in the present invention in an aqueous solution is shown below.

### 1. Test method

Injectable distilled water was added to 1 g of the 2Na salt of the compound (1) to prepare 100 mL of a sample solution. The sample solution was equally divided. One portion was shielded against light, and the other portion was unshielded. Each of them was stored at 35°C for 11 days during which these sample solutions were assayed over time using high-performance liquid chromatography and examined for stability.

### Measurement conditions for high-performance liquid chromatography

YMC-Pack ODS-AA-302 (YMC Co., Ltd.) was used as a column, and the column temperature was kept at a constant temperature around 40°C. Atetrahydrofuran/0.1% phosphoric acid (54:46) mixed solution was used as a mobile phase. A flow rate was adjusted such that the retention time of the compound (1) was approximately 7 minutes. An ultraviolet absorptiometer (measurement wavelength: 265 nm) was used as a detector.

### 2. Results

The assay results are shown in Tables 3 and 4. The aqueous solution containing the 2Na salt of the compound (1) was stable at 35°C for 11 days under light-shielded or unshielded conditions.

**[Table 3]**

| Table 3 Stability under light-shielded conditions | | | | |
|---|---|---|---|---|
| The number of days elapsed | 0 | 1 | 6 | 11 |
| Purity (%) | 99.8 | 99.8 | 99.8 | 99.8 |

**[Table 4]**

| Table 4 Stability under unshielded conditions | | | | |
|---|---|---|---|---|
| The number of days elapsed | 0 | 1 | 6 | 11 |
| Purity (%) | 99.8 | 99.8 | 99.7 | 99.7 |

### Example 3

Typical preparation examples used in the present invention are shown below.

### Preparation Example 1

### Production of tablets

| | |
|---|---|
| Propanedioic acid derivative of the present invention | 10.0 g |
| Lactose | 9.0 g |
| Hydroxypropylcellulose | 2.0 g |
| Crystalline cellulose | 7.7 g |
| Magnesium stearate | 0.3 g |
| Talc | 1.0 g |

These ingredients are prepared into tablets containing 100 mg of the propanedioic acid derivative of the present invention by a routine method.

### Preparation Example 2

### Production of injections

2.8 g of the propanedioic acid derivative of the present invention is dissolved in 4000 mL of distilled water. Dust is removed from the solution using a dust filter, and the resulting solution is sterilized using a 0.2-µm sterilization filter. The solution is charged into vials in an amount of 1 mL/vial and freeze-dried by a routine method to obtain 4000 ampules of injections containing 0.7 mg of the compound of the present invention per ampule. These preparations are used by adding 1 mL of injectable distilled water to each ampule.

### Preparation Example 3

### Production of powders for inhalation

5.6 g of the propanedioic acid derivative of the present invention and 400 g of maltose were dissolved in 4000 mL of distilled water. Then, the solution was freeze-dried by a routine method and pulverized to obtain powders for inhalation.

### Example 4

### Prophylactic or therapeutic effect of propanedioic acid derivative of the present invention on pulmonary fibrosis

The present inventors conducted a test using mouse models with pulmonary fibrosis induced by transtracheally administering bleomycin, for the purpose of studying the antifibrotic action of the propanedioic acid derivative of the present invention *in vivo.* This experimental model of pulmonary fibrosis is clinically similar to idiopathic pulmonary fibrosis in pathological observations such as increased inflammatory cells and the increased amount of hydroxyproline in an extracellular matrix-constituting collagen ingredient used as an index for fibrosis, and is widely used.

Acute inflammatory reaction occurs by 8 days after bleomycin administration. Subsequently, extracellular matrix accumulates due to change in fibrin formation and distorts the lung structure. Around 9 days after bleomycin administration, inflammation is allegedly switched to fibrosis (The International Journal of Biochemistry & Cell Biology Vol. 40, No. 3, Page. 362-382 (2008)).

There are many reports on drugs that exhibited antifibrotic effect by administration to bleomycin-induced pulmonary fibrosis models at an inflammatory stage or from an inflammatory stage. However, there are very few reports on drugs that exhibited antifibrotic effect by administration from a fibrotic stage (The International Journal of Biochemistry & Cell Biology Vol. 40, No. 3, Page. 362-382 (2008)).

### Test Example 1

### In vivo effect on pulmonary fibrosis induced by bleomycin

### 1. Test substance

A 2Na salt of a compound (1) (disodium salt of [5-[[6-[4-phenyl-6-(phenylmethoxy)-2-pyrimidinyl]-2-naphthalenyl]oxy]pentyl]propanedioic acid) of the present invention was used as a test substance.

### 2. Test method

C57BL/6 mice (female, 12 week old, body weight: 20 to 23 g) were anesthetized by the intraperitoneal administration of pentobarbital. The trachea was incised, and bleomycin (NIPPON KAYAKU CO., LTD.) (1.5 mg/kg) dissolved in saline was transtracheally administered thereto.

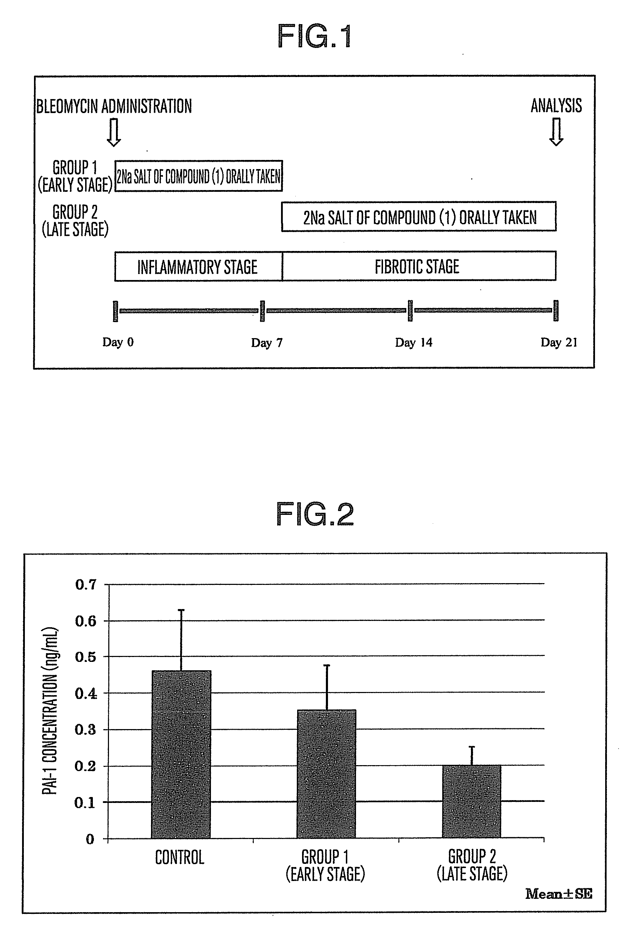

Test substance administration groups were classified into 2 groups, and 6 mice were used in each group. An aqueous solution containing 1000 ppm 2Na salt of the compound (1) dissolved therein was administered as drinking water to group 1 over 7 days after bleomycin administration (i.e., a period in which inflammation was induced by bleomycin administration: inflammatory stage). The aqueous solution containing 1000 ppm 2Na salt of the compound (1) dissolved therein was administered as drinking water to group 2 from 8 days to 20 days after bleomycin administration (i.e., a period in which fibrosis was induced by bleomycin administration: fibrotic stage). Each group orally took the aqueous solution at a daily dose of 3 to 4 g on average. The amount of the 2Na salt of the compound (1) orally taken was approximately 20 mg/kg body weight/day/mouse on average. The administration schedule is shown in Fig. 1.

On the other hand, 6 mice were used in a control group, to which only bleomycin was administered without administering the test substance. Moreover, 3 mice were used in a normal group, to which neither bleomycin nor the test substance was administered. All procedures of sample collection from the mice were carried out after euthanasia using CO₂

20 days after bleomycin administration, a bronchoalveolar lavage fluid (BALF) was collected from each mouse, and a PAI-1 concentration (ng/mL) in the fluid was measured using an ELISA kit (Mouse PAI-1 ELISA kit, Innovative research Inc.). Subsequently, the amount of hydroxyproline was measured for pulmonary fibrosis evaluation. The amount of hydroxyproline in the lung tissue was measured as the amount in a lung tissue hydrolysate according to the method of Kivirikko et al. (Anal. Biochem. 19, 249-255 (1967)). The Mann-Whitney U test was used in statistical analysis.

### 3. Results

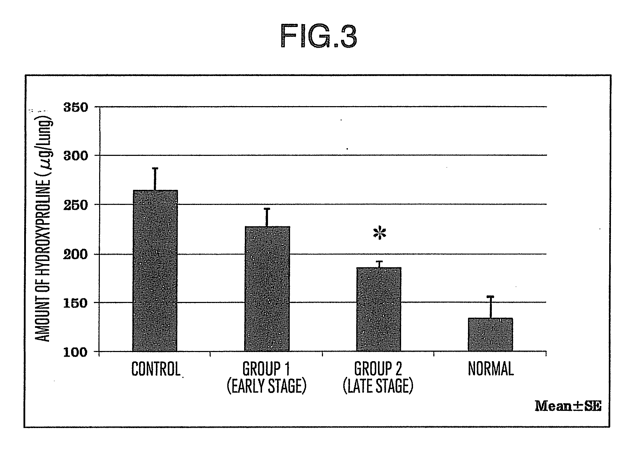

A graph of the PAI-1 concentration (ng/mL) in the bronchoalveolar lavage fluid (BALF) from each group is shown in Fig. 2.
A graph of the amount of hydroxyproline from each group is shown in Fig. 3. It was shown that the amount of hydroxyproline was significantly decreased in group 2 (late stage) that received the 2Na salt of the compound (1) only at the fibrotic stage, compared with the control group. This result demonstrated that the 2Na salt of the compound (1) having PAI-1 inhibitory effect suppressed the fibrotic process of the lung induced by bleomycin.

The 2Na salt of the compound (1) was mixed with drinking water and orally administered not by a forced manner at a regular basis but by free feeding. The 2Na salt of the compound (1) exhibited remarkable effectiveness that suppressed the fibrotic process of the lung induced by bleomycin, irrespective of the amount of drinking water taken once and the number of intakes of drinking water. Such effect is ascribable to the specific *in vivo* pharmacokinetics of the propanedioic acid derivative of the present invention.

In this way, a period for 7 days after bleomycin administration is defined as an inflammatory stage in which lung inflammation is induced, and a period from 8 days after bleomycin administration to the subsequent days is defined as a fibrotic stage in which fibrosis is induced. The propanedioic acid derivative of the present invention was administered at the inflammatory and fibrotic stages and evaluated by measuring the amount of hydroxyproline. As a result, it was confirmed that the amount of hydroxyproline was significantly decreased at the fibrotic stage, demonstrating that the propanedioic acid derivative of the present invention remarkably suppressed the fibrotic process of the lung.

A clinical concern about pulmonary fibrosis is the sustained progression of the fibrotic process. The propanedioic acid derivative of the present invention exhibited antifibrotic action by administration to bleomycin-induced pulmonary fibrosis models from the pulmonary fibrotic stage. This means a high possibility of suppression or at least prolonged onset of this sustainedly progressing fibrosis and suggests a possibility of high clinical usefulness.

From these test results, it was newly found that the propanedioic acid derivative of the present invention is exceedingly effective as a prophylactic or therapeutic agent for pulmonary fibrosis.

### Industrial Applicability

A propanedioic acid derivative of the present invention having PAI-1 inhibitory activity can be used in the prevention or treatment of pulmonary fibrosis and is useful as a prophylactic or therapeutic agent for pulmonary fibrosis.

## Claims

1. A pharmaceutical composition for preventing or treating pulmonary fibrosis, comprising a propanedioic acid derivative represented by a following formula (1) or a pharmaceutically acceptable salt thereof as an active ingredient: 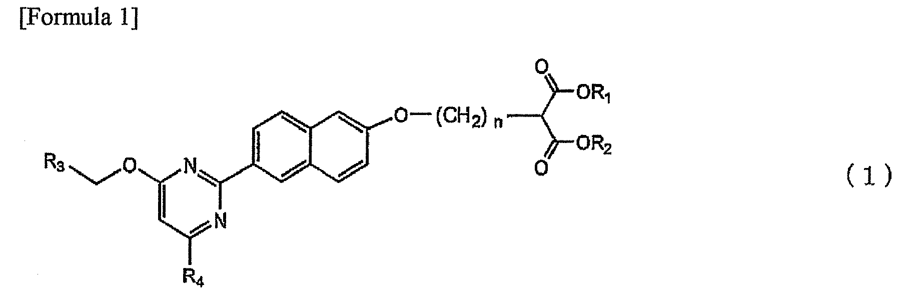 wherein
R₁ and R₂ each independently represent a hydrogen atom or a linear or branched alkyl group having 1 to 4 carbon atoms;
R₃ represents a cyclohexyl group or a phenyl group (wherein the phenyl group may be substituted by a substituent selected from a linear or branched alkyl group having 1 to 4 carbon atoms, a linear or branched alkoxy group having 1 to 4 carbon atoms, and a halogen atom);
R₄ represents a linear or branched alkyl group having 1 to 6 carbon atoms or a phenyl group (wherein the phenyl group may be substituted by a substituent selected from a linear or branched alkyl group having 1 to 4 carbon atoms, a linear or branched alkoxy group having 1 to 4 carbon atoms, and a halogen atom); and
n represents an integer of 1 to 8.

2. The pharmaceutical composition according to claim 1, wherein the propanedioic acid derivative or the pharmaceutically acceptable salt thereof is a propanedioic acid derivative represented by a following formula (2) or a pharmaceutically acceptable salt thereof: 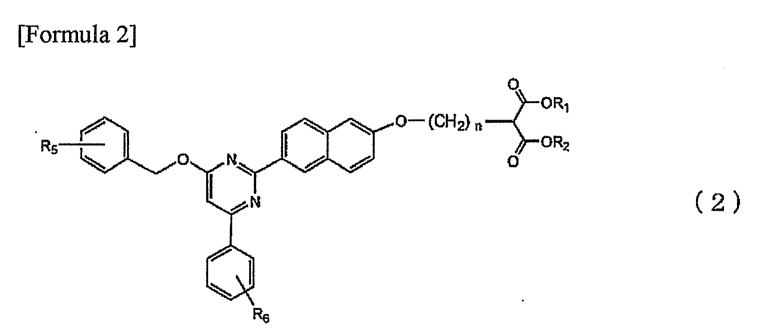 wherein
R₁, R₂, and n are as defined above;
R₅ represents a hydrogen atom or a halogen atom; and R₆ represents a hydrogen atom or a linear or branched alkoxy group having 1 to 4 carbon atoms.

3. The pharmaceutical composition according to claim 1 or 2, wherein the propanedioic acid derivative or the pharmaceutically acceptable salt thereof is a propanedioic acid derivative selected from a following group of compounds or a pharmaceutically acceptable salt thereof:
[5-[[6-[4-phenyl-6-(phenylmethoxy)-2-pyrimidinyl]-2-naphthalenyl]oxy]pentyl]propanedioic acid,
[3-[[6-[4-[(2-fluorophenyl)methoxy]-6-phenyl-2-pyrimidinyl]-2-naphthalenyl]oxy]propyl]propanedioic acid,
[3-[[6-[4-[(2-fluorophenyl)methoxy]-6-(4-methoxyphenyl)-2-pyrimidinyl]-2-naphthalenyl]oxy]propyl]propanedioic acid,
[5-[[6-[4-(cyclohexylmethoxy)-6-(1,1-dimethylethyl)-2-pyrimidinyl]-2-naphthalenyl]oxy]pentyl]propanedioic acid,
[5-[[6-[4-(1,1-dimethylethyl)-6-[(4-fluorophenyl)methoxy]-2-pyrimidinyl]-2-naphthalenyl]oxy]pentyl]propanedioic acid, and
[5-[[6-[4-[(2-chlorophenyl)methoxy]-6-(1,1-dimethylethyl)-2-pyrimidinyl]-2-naphthalenyl]oxy]pentyl]propanedioic acid.

4. The pharmaceutical composition according to any one of claims 1 to 3, wherein the propanedioic acid derivative or the pharmaceutically acceptable salt thereof is [5-[[6-[4-phenyl-6-(phenylmethoxy)-2-pyrimidinyl]-2-naphthalenyl]oxy]pentyl]propanedioic acid or a pharmaceutically acceptable salt thereof.

5. The pharmaceutical composition according to any one of claims 1 to 4, comprising a pharmaceutically acceptable carrier and/or excipient together with the propanedioic acid derivative or the pharmaceutically acceptable salt thereof.

6. Use of a propanedioic acid derivative or a pharmaceutically acceptable salt thereof according to any one of claims 1 to 4 for producing a prophylactic or therapeutic agent for pulmonary fibrosis.

7. A method for preventing or treating pulmonary fibrosis, comprising administering a propanedioic acid derivative or a pharmaceutically acceptable salt thereof according to any one of claims 1 to 4 to a human or a nonhuman mammal.
